# EUROPEAN PATENT APPLICATION

(11) **EP 4 582 081 A1**
(43) Date of publication of application: **09.07.2025**
(21) Application number: 23859368.5
(22) Date of filing: 30.08.2023
(51) Int. Cl.: A61K 9/10, A61K 9/72, A61K 31/137, A61K 31/4704, A61K 31/538, A61K 31/56, A61K 45/06, A61P 11/06, A61P 11/00

(54) **PHARMACEUTICAL FORMULATION COMPRISING COMBINATION OF LONG-ACTING INHALED STEROID AND LONG-ACTING BETA2 RECEPTOR AGONIST**

(30) Priority: 30.08.2022 CN 202211044167
(71) Applicant: Lexenpharm (Suzhou) Limited, Suzhou, Jiangsu 215123 (CN)
(72) Inventor: LEE, Junning, Suzhou, Jiangsu 215123 (CN); DI, Yingfeng, Suzhou, Jiangsu 215123 (CN)
(74) Representative: Schulz Junghans Patentanwälte PartGmbB
(86) International application number: PCT/CN2023/115647
(87) International publication number: WO 2024/046339

(57) **Abstract**

The present invention discloses a pharmaceutical formulation comprising a combination of long-acting inhaled steroid and long-acting β2 receptor agonist, and relates to the technical field of pharmaceutical formulation. The long-acting inhaled steroid drug is selected from fluticasone or pharmaceutically acceptable salts; the β2 receptor agonist is selected from vilanterol, olodaterol, indacaterol, or any pharmaceutically acceptable salt, isomer, or hydrate thereof; the dosage form of the pharmaceutical formulation is a suspension or co-suspension. The present invention significantly improves the stability of the prepared pharmaceutical formulation by controlling the type and preparation method of drug excipients. **In** addition, the present invention also discloses the use of the pharmaceutical formulation in the preparation of pharmaceutical composition for inhalation for the prevention or treatment of respiratory diseases such as asthma and chronic obstructive pulmonary disease.

## Description

The present invention claims priority to a Chinese patent application filed with the Chinese Patent Office on August 30, 2022, with application number 202211044167.9 and the title of the invention "Pharmaceutical Preparation Comprising Combination of Long-Acting Inhaled Steroid and Long-Acting β2 Receptor Agonist", the entire contents of which are incorporated herein by reference.

### FIELD OF THE INVENTION

The present invention pertains to the field of pharmaceutical formulation and specifically relates to a pharmaceutical formulation comprising a combination of a long-acting inhaled steroid and a long-acting β2 receptor agonist.

### BACKGROUND OF THE INVENTION

Chronic obstructive pulmonary disease (COPD) is a common chronic respiratory disease with a large number of patients and a high mortality rate, which affects public health and imposes a heavy economic burden on society. COPD is mainly characterized by irreversible airflow limitation, and the condition usually progressively worsens, which is related to the abnormal inflammatory response of lung tissue to harmful particles or gases. Due to the high frequency of inhalation and complex devices, treatment compliance has become a common medical challenge for COPD and asthma patients. Therefore, the new generation of long-acting β2 receptor agonists (LABA) that can quickly take effect and have a broncho-dilating effect that lasts for 24 hours is expected to improve treatment compliance.

Currently, therapies for treating or preventing COPD and asthma include the use of long-acting bronchodilators or inhaled corticosteroids (ICS).

Inhaled bronchodilators are the foundation of COPD and asthma therapy, as they can alleviate symptoms, reduce disease attacks, and improve quality of life. These drugs can also improve airflow limitation and overinflation, thereby reducing respiratory activity and improving exercise tolerance. Moreover, bronchodilators can reduce respiratory muscle fatigue and improve mucociliary clearance function.

Bronchodilators include β2 agonists, which may be short-acting for immediate relief of asthma symptoms, or long-acting for long-term prevention of asthma symptoms.

Long-acting β2-agonist (LABA) or ultra-long-acting β2-agonist (ULABA) can improve lung function, reduce symptoms, and prevent exercise-induced respiratory distress in patients with asthma and COPD. LABA causes bronchial dilation by inducing the elongation and relaxation of airway smooth muscle. Secondly, LABA also plays other roles, such as inhibiting the proliferation of airway smooth muscle cells and the release of inflammatory mediators, as well as non-smooth muscle effects, such as stimulating mucosal ciliary transport function, cellular protection of respiratory mucosa, and weakening neutrophil recruitment and activation.

In addition, using LABA can reduce the frequency of medication intake. Commercially available twice daily LABAs include salmeterol, formoterol and Afamoterol. The commercially available once daily ultra-long-acting β2-agonist ULABA include indacaterol, vilanterol, camoterol and olodaterol.

Although β2-agonists and anticholinergic drugs provide symptom relief for bronchoconstriction, another element of COPD inflammation requires individual treatment with corticosteroids, and most inhaled corticosteroids require a multiple-dosage regimen.

Corticosteroids have shown inhibitory effects on inflammatory cells and mediators involved in the pathology of respiratory diseases such as COPD. The use of corticosteroid/glucocorticoid treatment is considered the most promising and effective therapy currently available for COPD.

There are several commercially available inhalation pharmaceutical compositions comprising LABA and ICS. The examples used for asthma and COPD are dry powder inhaler (DPI) and metered-dose inhaler (MDI) such as salmeterol / fluticasone propionate (Advair^{®} diskus and Advair^{®} HFA) and formoterol fumarate dihydrate / budesonide (Symbicort^{®}); formoterol and mometasone (Dulera^{®}); salmeterol / fluticasone furoate (AirDuo^{™} Respiclick^{®}); valantro and fluticasone furoate (Breo Ellipta^{®}), or the like.

The structural formula for fluticasone furoate is as follows:

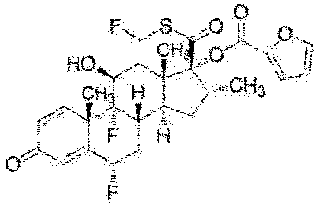

Fluticasone furoate is a novel synthetic fluorinated glucocorticoid, which was approved by the FDA in April 2007 for the treatment of seasonal and perennial allergic rhinitis. As a rapidly acting and potent glucocorticoid, it has become a classic treatment for allergic rhinitis. Compared with fluticasone propionate, fluticasone furoate has a higher receptor affinity; the transport thereof from glucocorticoid receptors to the nucleus is faster, and the nuclear retention time thereof is also longer; the selectivity thereof exceeds that of other glucocorticoids; the reduced extracellular flow and increased intracellular binding thereof result in its prolonged duration of drug efficacy. Compared with other glucocorticoids, fluticasone furoate has the strongest effect in maintaining epithelial integrity and reducing epithelial permeability in response to protease or mechanical cell damage.

The structural formula for vilanterol trifenatate is as follows:

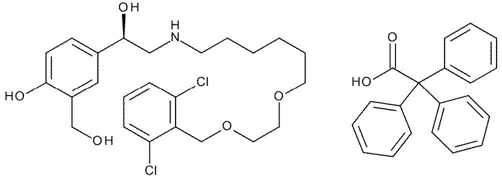

Vilanterol trifenatate is a novel oral administration long-acting selective β2 receptor agonist, which has a faster onset and longer duration of action compared to salmeterol. In addition, the affinity of vilanterol trifenatate for β2 receptors is 1000 times higher than that for J31 receptors, showing a high selectivity. Clinical studies have shown that the broncho-dilating effect of vilanterol trifenatate can last for 24 hours. Continuous daily treatment of vilanterol trifenatate for 4 weeks can significantly improve lung function in COPD patients with good tolerability and safety, and the incidence of adverse reactions such as LABA effects (increased blood glucose levels, hypertension, decreased blood potassium levels, tremors, and palpitations) is also low.

The structural formula for olodaterol hydrochloride is as follows:

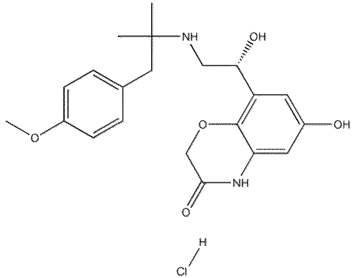

Olodaterol hydrochloride is an ultra-long acting β2-adrenergic receptor agonist. Due to its longer half-life, it can be administered once daily, and thus it is expected to improve patient treatment compliance. Olodaterol hydrochloride was developed by Boehringer Ingelheim and launched in the United States in 2014 under the brand name Striverdi^{®} for the single drug and the brand name Spiolto^{®} for the combination drug. It is a novel selective and rapid-acting LABA with high selectivity for β2-adrenergic receptors, rapid onset and long half-life of more than 12 hours, and it can maintain the broncho-dilating effect for 24 hours. Therefore, it can be administered once a day.

The structural formula for indacaterol maleate is as follows.

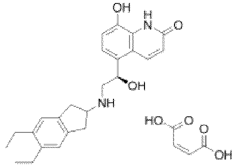

Indacaterol maleate, as a bronchodilator, belongs to LABA and is suitable for maintenance therapy in adult patients with COPD. Indacaterol has the characteristic of being effective for 5 minutes and lasting for 24 hours, so it can be administered once a day. Indacaterol has been produced by Novartis International AG in Switzerland and has been marketed in over 70 countries and regions worldwide since 2009. Approved for listing in China by the National Medical Products Administration in June 2012 under the brand name Onbrez^{®}. It is the first single agent LABA approved for the treatment of COPD in China.

U.S. patent US 11,116,721 B2 discloses a combination dry powder inhalation drug comprising the long-acting β2 agonist vilanterol trifenatate and the long-acting steroid fluticasone furoate. Chinese patent CN 104470503 A discloses a combination dry powder inhalation drug comprising the long-acting β2 agonist vilanterol trifenatate, the long-acting steroid fluticasone furoate and umeclidinium bromide. Chinese patent CN 103501776 A discloses a combination dry powder inhalation drug comprising glycyrrhizinate, vilanterol and fluticasone furoate.

Based on the prior arts mentioned above, it can be concluded that the current pharmaceutical compositions are mostly dry powder inhalation drugs and aerosols. The use of such drugs requires patients to hold their breath or have sufficient inspiratory flow, as well as the ability to coordinate their hands and breathing. Therefore, they cannot meet the clinical needs of children with severe and extremely severe asthma and COPD. However, most of the pharmaceutical compositions on the market are administered multiple times a day, and the patient's medication compliance is low. Currently, there is no once-a-day ICS/LABA ultra long effect nebulized inhalation suspension preparation. Therefore, it is necessary to provide a treatment scheme for patients who cannot use portable dry powder inhaler (DPI), metered dose inhaler (MDI) and soft mist inhaler (SMI), and provide patients at all stages with a once-a-day sterile inhalation suspension comprising a combination of long-acting inhaled steroid and long-acting β2-agonist to treat asthma and COPD.

### SUMMARY OF THE INVENTION

In view of the existing problems in the prior art, one objective of the present invention is to provide a pharmaceutical formulation comprising a combination of long-acting inhaled steroid and long-acting β2-agonist; wherein the pharmaceutical formulation can be administered once a day to treat asthma and COPD.

The long-acting inhaled steroid drug is selected from fluticasone or a pharmaceutically acceptable salt of fluticasone.

Preferably, the long-acting inhaled steroid drug is fluticasone furoate;
the β2 receptor agonist is selected from vilanterol, olodaterol, indacaterol, or any pharmaceutically acceptable salt, isomer, or hydrate thereof;
preferably, the vilanterol is in the form of vilanterol trifenatate, the olodaterol is in the form of olodaterol hydrochloride, and the indacaterol is in the form of indacaterol maleate salt;
preferably, the long-acting β2-agonist is selected from one or more of vilanterol trifenatate, olodaterol hydrochloride, and indacaterol maleate.

The dosage form of the pharmaceutical formulation is suspension or co-suspension; the pH of the pharmaceutical formulation is 2.5-6.0.

In the suspension or co-suspension, at least 90% of the active agent particles have an optical diameter of 7 µm or less by volume; at least 50% of the active agent particles by volume have an optical diameter of 5 µm or less by volume.

As a preferred embodiment, the pharmaceutical formulation comprises a compound suspension of fluticasone furoate or a physiologically acceptable salt of fluticasone furoate in combination with vilanterol;
preferably, the pharmaceutical formulation is a nebulized inhalation co-suspension formulation comprising vilanterol and fluticasone furoate;
the vilanterol is vilanterol containing a triphenylacetate salt form dissolved in water phase;
the pharmaceutical formulation is a nebulized inhalation co-suspension comprising vilanterol trifenatate and fluticasone furoate.

In the pharmaceutical formulation, the daily inhalation dose of vilanterol trifenatate is 10-100 µg, and the daily inhalation dose of fluticasone furoate is 25-1000 µg.

In some preferred embodiments, the nebulized inhalation suspension formulation of vilanterol trifenatate and fluticasone furoate further comprises sulfobutyl-β-cyclodextrin, with 5mg-500 mg of sulfobutyl-β-cyclodextrin per ml of liquid formulation.

As another preferred embodiment, the pharmaceutical formulation comprises a compound suspension of fluticasone furoate or a physiologically acceptable salt of fluticasone furoate in combination with olodaterol;
preferably, the pharmaceutical formulation is a nebulized inhalation suspension formulation comprising olodaterol and fluticasone furoate;
the olodaterol is olodaterol containing a hydrochloride salt form dissolved in the aqueous phase;
the pharmaceutical formulation is a nebulized inhalation suspension comprising olodaterol hydrochloride and fluticasone furoate.

In the pharmaceutical formulation, the daily inhalation dose of olodaterol hydrochloride is 1-100 µg, and the daily inhalation dose of fluticasone furoate is 25-1000 µg.

As a further preferred embodiment, the pharmaceutical formulation comprises a compound co-suspension of fluticasone furoate or a physiologically acceptable salt of fluticasone furoate in combination with indacaterol maleate;
preferably, the pharmaceutical formulation is a nebulized inhalation co-suspension formulation comprising indacaterol and fluticasone furoate;
the indacaterol is indacaterol containing maleic acid salt form dissolved in water phase;
the pharmaceutical formulation is a nebulized inhalation co-suspension comprising indacaterol maleate and fluticasone furoate.

In the pharmaceutical formulation, the daily inhalation dose of indacaterol maleate is 20-200 µg, and the daily inhalation dose of fluticasone furoate is 25-500 µg.

The pharmaceutical formulation is a pharmaceutical formulation for inhalation;
the administration method for the pharmaceutical formulation is spray administration;
the pharmaceutical formulation further comprises one or more of isotonic agents, buffering agents, excipients, stabilizers, and chelating agents;
the isotonic agent is sodium chloride;
the buffering agent is citric acid or phosphate buffering agent;
the excipient is selected from one or more of polysorbate 20, polysorbate 80, sorbitan monolaurate, poloxamer, polyoxyethylene castor oil, polyethylene glycol, solutol HS15, and polyvinylpyrrolidone, preferably polysorbate 80.

The stabilizer is selected from one or more of ethylenediaminetetraacetic acid, dehydrated disodium ethylenediaminetetraacetate, disodium ethylenediaminetetraacetate, and citric acid; preferably ethylenediaminetetraacetic acid.

The present invention further provides a method for preparing the above-mentioned pharmaceutical formulation, comprising the following steps:
(1) adding formulated amounts of excipients to water for injection and stirring to dissolve, adjusting the pH with buffer solution, and preparing a sterile buffer solution by filtering through a 0.22 µm filter membrane;
(2) dispersing a raw material of the long-acting inhaled steroid sterilized by dry heat in the aforementioned 10% volume buffer solution by a dispenser, to obtain a dispersion of the raw material of the long-acting inhaled steroid; and adding the β2 receptor agonist to a certain amount of buffer solution, and after specific processing, filtering through a 0.22 µm membrane, to obtain a dispersion of β2 receptor agonist;
(3) mixing the dispersions of the two raw materials with a remaining buffer solution, and dispersing with a disperser, to prepare a sterile suspension or co-suspension; and
(4) filling the suspension into a low-density polypropylene (LDPE) bottle to prepare a sterile suspension or co-suspension for inhalation.

The present invention also provides the use of the above-mentioned pharmaceutical formulation in the preparation of a medicament for treating respiratory diseases, wherein the respiratory diseases include but are not limited to chronic obstructive pulmonary disease (COPD) and all types of asthma.

Compared with the prior art, the beneficial effects of the invention are:
(1) The pharmaceutical formulations prepared by prior art comprising a long-acting inhaled steroid and a long-acting β2 receptor agonist are mostly dry powder inhalations or aerosols. The use of such drugs requires patients to hold their breath or have sufficient inspiratory flow, as well as the ability to coordinate their hands and breathing. Therefore, they cannot meet the clinical needs of children with severe and extremely severe asthma and COPD patients. The present invention provides a suspension or co-suspension comprising a combination of a long-acting inhaled steroid and a long-acting β2 receptor agonist, which can quickly take effect through nebulized inhalation, reduce systemic drug exposure, reduce off-target effect, thereby providing a reliable treatment solution for patients of any age or who cannot use portable dry powder inhaler (DPI), metered dose inhaler (MDI) and soft mist inhaler (SMI);
(2) In the implementation of the present invention, by reasonably controlling drugs, by controlling the type and amount of drug excipients, as well as by controlling the particle size of the drug components, and controlling the pH of the pharmaceutical formulation to 2.5-6.0, the stability of the prepared suspension or co-suspension is significantly improved., the single maximum impurity and total impurity content in the pharmaceutical formulation will not increase significantly after long-term storage or high-temperature storage, and the pharmaceutical formulation has good dispersibility.
(3) In traditional processes, long-acting β2 receptor agonists such as vilanterol and indacaterol are insoluble in water and thus cannot be filtered for sterilization, and are prone to degradation under high-temperature conditions and thus cannot be sterilized at high temperatures. During the implementation of the present invention, specific processes were used to achieve filtration and sterilization of vilanterol and indacaterol, and a once-a-day sterile inhalation suspension or co-suspension comprising a long-acting inhaled steroid and a long-acting β2 receptor agonist was finally formed.

### BRIEF DESCRIPTION OF THE DRAWINGS

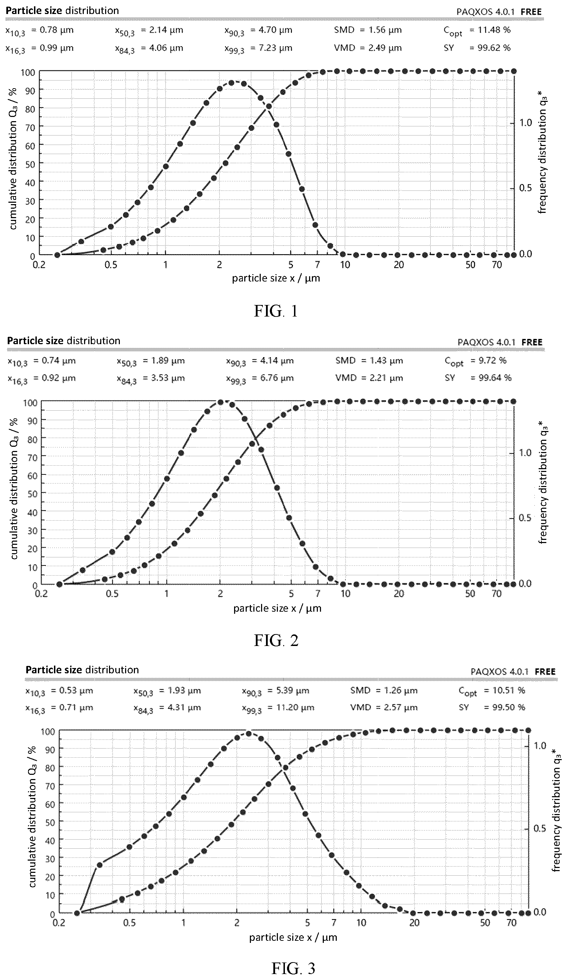

FIG. 1 is the particle size distribution diagram of sample 1 in test example 1;
FIG. 2 is the particle size distribution diagram of sample 2 in test example 1;
FIG. 3 is the particle size distribution diagram of sample 3 in test example 1;
FIG. 4 is a graph showing the changes in the total impurity of fluticasone furoate-vilanterol trifenatate co-suspension in different pH ranges at 60°C for 10 days;
FIG. 5 is a graph showing the changes in the total impurity of fluticasone furoate-vilanterol trifenatate-cyclodextrin suspension in different pH ranges at 60°C for 10 days;
FIG. 6 is a graph showing the changes in the total impurity of fluticasone furoate-olodaterol hydrochloride suspension in different pH ranges at 60°C for 10 days;
FIG. 7 is a graph showing the changes in the total impurity of fluticasone furoate-indacaterol maleate co-suspension in different pH ranges at 60°C for 10 days.

### DETAILED DESCRIPTION OF THE EMBODIMENTS

The technical solution of the present invention is further defined below in conjunction with specific embodiments, but the claimed scope is not limited to the description made.

The supplier and model of the components used in the present invention are as follows:
fluticasone furoate: supplier: Aurisco, Batch No.: A28-180801F13
vilanterol trifenatate: supplier: Inke, Batch No.: PP-6M
olodaterol hydrochloride: supplier: Inke, Batch No.: PP-1
indacaterol maleate: supplier: Inke, Batch No.: P-4

The supplier and model of the equipment used in the present invention are as follows:
disperser: supplier: IKA, Model: T18
homogenizer, supplier: PSI, Model: PSI 20

### EXAMPLE 1 Fluticasone furoate-vilanterol trifenatate co-suspension for inhalation and the method for preparing thereof

The components of the single vial and the total amount of formulation of fluticasone furoate-vilanterol trifenatate co-suspension for inhalation are shown in the table below.

| component | single vial formulated amount | total amount |
|---|---|---|
| fluticasone furoate raw material drug | 0.17mg | 0.085 g |
| vilanterol trifenatate raw material drug | 0.068mg | 0.034g |
| polysorbate 80 | 0.4mg | 0.2g |
| EDTA | 0.2mg | 0.1g |
| sodium chloride | 17mg | 8.5 g |
| water for injection | *q.s.* to 2 mL | *q.s.* to 1000 mL |

The preparation process:
(1) adding the formulated amount of excipients to 800 ml of water for injection, stirring to dissolve and making up to 1000 ml, to prepare a buffer solution;
(2) dispersing the fluticasone furoate raw material in the above 100 ml buffer solution by disperser at a dispersing speed of 9000 rpm for a dispersion time of 30 min, and then adding the vilanterol trifenatate raw material, continue dispersing by the disperser at 9000 rpm for 30 min, to obtain a dispersion of the two raw materials;
(3) mixing the dispersion of the raw materials with the remaining buffer solution and dispersing by the disperser at 9000 rpm for 30 min to prepare a co-suspension; and
(4) filling the above-mentioned suspension into a 2 ml LDPE bottle to prepare the fluticasone furoate-vilanterol co-suspension for inhalation.

### EXAMPLE 2 Preparation of fluticasone furoate single suspension for inhalation

The components of the single vial and the amount of formulation of fluticasone furoate single suspension for inhalation are shown in the table below.

| component | single vial formulated amount | total amount |
|---|---|---|
| fluticasone furoate raw material drug | 0.17mg | 0.085 g |
| polysorbate 80 | 0.4mg | 0.2g |
| EDTA | 0.2mg | 0.1g |
| sodium chloride | 17mg | 8.5 g |
| water for injection | *q.s.* to 2 mL | *q.s.* to 1000 mL |

The preparation process:
(1) adding the formulated amount of excipients to 800 ml of water for injection, stirring to dissolve and making up to 1000 ml, to prepare a buffer solution;
(2) dispersing the fluticasone furoate raw material in the above 100 ml buffer solution by disperser at a dispersing speed of 9000 rpm and dispersion time of 30 min;
(3) mixing the dispersion of the raw material with the remaining buffer solution and dispersing by the disperser at 9000 rpm for 30 min to prepare a co-suspension; and
(4) filling the above-mentioned suspension into a 2 ml LDPE bottle to prepare the fluticasone furoate single suspension for inhalation.

### EXAMPLE 3 Preparation of vilanterol trifenatate single suspension for inhalation

The components of the single vial and the amount of formulation of vilanterol trifenatate single suspension for inhalation are shown in the table below.

| component | single vial formulated amount | total amount |
|---|---|---|
| vilanterol trifenatate raw material drug | 0.068mg | 0.034g |
| polysorbate 80 | 0.4mg | 0.2g |
| EDTA | 0.2mg | 0.1g |
| sodium chloride | 17mg | 8.5 g |
| water for injection | *q.s.* to 2 mL | *q.s.* to 1000 mL |

The preparation process:
(1) adding the formulated amount of excipients to 800 ml of water for injection, stirring to dissolve and making up to 1000 ml, to prepare a buffer solution;
(2) dispersing the vilanterol trifenatate raw material in the above 100 ml buffer solution by disperser at a dispersing speed of 9000 rpm and dispersion time of 30 min;
(3) mixing the dispersion of the raw material with the remaining buffer solution and dispersing by the disperser at 9000 rpm for 30 min to prepare a co-suspension; and
(4) filling the above-mentioned suspension into a 2 ml LDPE bottle to prepare the vilanterol trifenatate single suspension for inhalation.

### TEST EXAMPLE 1 Particle sizes of the co-suspension and single suspensions prepared in Examples 1-3 measured by Sympatec laser particle size analyzer

Sample 1, Sample 2 and Sample 3 are respectively fluticasone furoate-vilanterol co-suspension (Example 1), fluticasone furoate single suspension (Example 2), and vilanterol single suspension (Example 3). The particle size distributions thereof are listed in Table 1 below.

**Table 1**

| No. | D10 | D50 | D90 |
|---|---|---|---|
| Sample 1 | 0.78µm | 2.14µm | 4.70µm |
| Sample 2 | 0.74µm | 1.89µm | 4.14µm |
| Sample 3 | 0.53µm | 1.93µm | 5.39µm |

### TEST EXAMPLE 2 Preparation and stability study of fluticasone furoate-vilanterol trifenatate co-suspensions in different pH ranges

Seven batches of co-suspension were prepared according to the formulation and method for preparation listed in example 1. 0.1 M citric acid-sodium citrate buffer with a pH of 3.0, 0.1 M citric acid-sodium citrate buffer with a pH of 6.6, and 1 M sodium citrate solution were prepared, and the pH values of the co-suspensions were adjusted to 3.0, 3.5, 4.0, 4.5, 5.0, 5.5, 6.0, 6.5 and 7.0 respectively by the solutions above. The high-temperature influence factor test (60°C for 10 days) was carried out to study the stability of the preparations in different pH ranges. The results of the study are shown in Table 2 below.

**Table 2**

| different groups of pH | T=0 | | | | T=10d 60°C | | | |
|---|---|---|---|---|---|---|---|---|
| | related substances (%) | fluticason e furoate | vilanterol trifenatate | redisper sibility | related substances (%) | fluticason e furoate | vilanterol trifenatate | redispersibility |
| 3.0 | total impurity | 1.3 | 1.20 | redisper sible | total impurity | 1.00 | 0.98 | redispersible |
| | maximum single impurity | 0.60 | 0.58 | | maximum single impurity | 0.38 | 0.40 | |
| 3.5 | total impurity | 0.39 | 0.40 | redisper sible | total impurity | 0.67 | 0.76 | redispersible |
| | maximum single impurity | 0.17 | 0.17 | | maximum single impurity | 0.21 | 0.21 | |
| 4.0 | total impurity | 0.27 | 0.53 | redisper sible | total impurity | 0.63 | 0.54 | redispersible |
| | maximum single impurity | 0.07 | 0.34 | | maximum single impurity | 0.24 | 0.18 | |
| 4.5 | total impurity | 0.18 | 0.63 | redisper sible | total impurity | 0.55 | 0.83 | redispersible |
| | maximum single impurity | 0.05 | 0.17 | | maximum single impurity | 0.21 | 0.38 | |
| 5.0 | total impurity | 0.17 | 0.23 | redisper sible | total impurity | 0.39 | 0.34 | redispersible |
| | maximum single impurity | 0.06 | 0.10 | | maximum single impurity | 0.20 | 0.14 | |
| 5.5 | total impurity | 0.35 | 0.47 | redisper sible | total impurity | 0.51 | 0.62 | few particles sedimented |
| | maximum single impurity | 0.21 | 0.19 | | maximum single impurity | 0.22 | 0.22 | |
| 6.0 | total impurity | 0.19 | 0.18 | redisper sible | total impurity | 0.43 | 0.81 | few particles sedimented |
| | maximum single impurity | 0.07 | 0.10 | | maximum single impurity | 0.23 | 0.24 | |
| 6.5 | total impurity | 0.36 | 0.52 | redisper sible | total impurity | 0.47 | 3.60 | agglomerates cannot be dispersed |
| | maximum single impurity | 0.20 | 0.18 | | maximum single impurity | 0.21 | 2.69 | |
| 7.0 | total impurity | 1.50 | 1.5 | redisper sible | total impurity | 0.70 | 5.80 | agglomerates cannot be dispersed |
| | maximum single impurity | 0.72 | 0.69 | | maximum single impurity | 0.28 | 4.03 | |

According to the data in Table 2 above, it can be seen that the fluticasone furoate-vilanterol trifenatate co-suspension has good stability at a pH of 3.5-6.0, especially the fluticasone furoate-vilanterol trifenatate co-suspension co-suspension has the best stability, low content of total impurity and maximum single impurity, and good dispersibility when the pH is 3.5-5.0.

### EXAMPLE 4 Fluticasone furoate-vilanterol trifenatate co-suspension for inhalation and the method for preparing thereof

The components of the single vial and the amount of formulation of fluticasone furoate-vilanterol trifenatate co-suspension for inhalation are: **fluticasone furoate-vilanterol trifenatate co-suspension for inhalation (pH 3.5) with 0.005% (w/w) EDTA / 0.01% (w/w) polysorbate 80 prepared in the example.**

| component | single vial formulated amount | total amount |
|---|---|---|
| fluticasone furoate raw material drug | 0.17mg | 0.085 g |
| vilanterol trifenatate raw material drug | 0.068mg | 0.034g |
| polysorbate 80 | 0.2mg | 0.1g |
| EDTA | 0.1mg | 0.05 g |
| sodium chloride | 17mg | 8.5 g |
| water for injection | *q.s.* to 2 mL | *q.s.* to 1000 mL |

The preparation method is:
(1) adding the formulated amount of excipients to 800 ml of water for injection, stirring to dissolve and making up to 1000 ml, to prepare a buffer solution, and adjusting pH to 3.5 by 0.1 M citric acid-sodium citrate buffer at pH 3.0;
(2) dispersing the fluticasone furoate raw material in the above 100 ml buffer solution by disperser at a dispersing speed of 9000 rpm for a dispersion time of 30 min, and then adding the vilanterol trifenatate raw material, continue dispersing by the disperser at 9000 rpm for 30 min, to obtain a dispersion of the two raw materials;
(3) mixing the dispersion of the raw materials with the remaining buffer solution and dispersing by the disperser at 9000 rpm for 30 min to prepare a co-suspension; and
(4) filling the above-mentioned suspension into a 2 ml LDPE bottle to prepare the fluticasone furoate-vilanterol co-suspension for inhalation.

### EXAMPLE 5 Fluticasone furoate-vilanterol trifenatate co-suspension for inhalation and the method for preparing thereof

The components of the single vial and the amount of formulation of fluticasone furoate-vilanterol trifenatate co-suspension for inhalation are: **fluticasone furoate-vilanterol trifenatate co-suspension for inhalation (pH 3.5) with 0.015% (w/w) EDTA / 0.01% (w/w) polysorbate 80 prepared in the example.**

| component | single vial formulated amount | total amount |
|---|---|---|
| fluticasone furoate raw material drug | 0.17mg | 0.085 g |
| vilanterol trifenatate raw material drug | 0.068mg | 0.034g |
| polysorbate 80 | 0.2mg | 0.1g |
| EDTA | 0.3mg | 0.15 g |
| sodium chloride | 17mg | 8.5 g |
| water for injection | *q.s.* to 2 mL | *q.s.* to 1000 mL |

The preparation method is:
(1) adding the formulated amount of excipients to 800 ml of water for injection, stirring to dissolve and making up to 1000 ml, to prepare a buffer solution, and adjusting pH to 3.5 by 0.1 M citric acid-sodium citrate buffer at pH 3.0;
(2) dispersing the fluticasone furoate raw material in the above 100 ml buffer solution by disperser at a dispersing speed of 9000 rpm for a dispersion time of 30 min, and then adding the vilanterol trifenatate raw material, continue dispersing by the disperser at 9000 rpm for 30 min, to obtain a dispersion of the two raw materials;
(3) mixing the dispersion of the raw materials with the remaining buffer solution and dispersing by the disperser at 9000 rpm for 30 min to prepare a co-suspension; and
(4) filling the above-mentioned suspension into a 2 ml LDPE bottle to prepare the fluticasone furoate-vilanterol co-suspension for inhalation.

### EXAMPLE 6 Fluticasone furoate-vilanterol trifenatate co-suspension for inhalation and the method for preparing thereof

The components of the single vial and the amount of formulation of fluticasone furoate-vilanterol trifenatate co-suspension for inhalation are: **fluticasone furoate-vilanterol trifenatate co-suspension for inhalation (pH 3.5) with 0.015% (w/w) EDTA / 0.03% (w/w) polysorbate 80 prepared in the example.**

| component | single vial formulated amount | total amount |
|---|---|---|
| fluticasone furoate raw material drug | 0.17mg | 0.085 g |
| vilanterol trifenatate raw material drug | 0.068mg | 0.034g |
| polysorbate 80 | 0.6mg | 0.3g |
| EDTA | 0.3mg | 0.15 g |
| sodium chloride | 17mg | 8.5 g |
| water for injection | *q.s.* to 2 mL | *q.s.* to 1000 mL |

The preparation method is:
(1) adding the formulated amount of excipients to 800 ml of water for injection, stirring to dissolve and making up to 1000 ml, to prepare a buffer solution, and adjusting pH to 3.5 by 0.1 M citric acid-sodium citrate buffer at pH 3.0;
(2) dispersing the fluticasone furoate raw material in the above 100 ml buffer solution by disperser at a dispersing speed of 9000 rpm for a dispersion time of 30 min, and then adding the vilanterol trifenatate raw material, continue dispersing by the disperser at 9000 rpm for 30 min, to obtain a dispersion of the two raw materials;
(3) mixing the dispersion of the raw materials with the remaining buffer solution and dispersing by the disperser at 9000 rpm for 30 min to prepare a co-suspension; and
(4) filling the above-mentioned suspension into a 2 ml LDPE bottle to prepare the fluticasone furoate-vilanterol co-suspension for inhalation.

### EXAMPLE 7 Fluticasone furoate-vilanterol trifenatate co-suspension for inhalation and the method for preparing thereof

The components of the single vial and the amount of formulation of fluticasone furoate-vilanterol trifenatate co-suspension for inhalation are: **fluticasone furoate-vilanterol trifenatate co-suspension for inhalation (pH 6.5) with 0.005% (w/w) EDTA / 0.01% (w/w) polysorbate 80 prepared in the example.**

| component | single vial formulated amount | total amount |
|---|---|---|
| fluticasone furoate raw material drug | 0.17mg | 0.085 g |
| vilanterol trifenatate raw material drug | 0.068mg | 0.034g |
| polysorbate 80 | 0.2mg | 0.1g |
| EDTA | 0.1mg | 0.05 g |
| sodium chloride | 17mg | 8.5 g |
| water for injection | *q.s.* to 2 mL | *q.s.* to 1000 mL |

The preparation method:
(1) adding the formulated amount of excipients to 800 ml of water for injection, stirring to dissolve and making up to 1000 ml, to prepare a buffer solution, and adjusting pH to 6.5 by 0.1 M citric acid-sodium citrate buffer at pH 6.6;
(2) dispersing the fluticasone furoate raw material in 100 ml buffer solution by disperser at a dispersing speed of 9000 rpm for a dispersion time of 30 min, and then adding the vilanterol trifenatate raw material, continue dispersing by the disperser at 9000 rpm for 30 min, to obtain a dispersion of the two raw materials;
(3) mixing the dispersion of the raw materials with the remaining buffer solution and dispersing by the disperser at 9000 rpm for 30 min to prepare a co-suspension; and
(4) filling the above-mentioned suspension into a 2 ml LDPE bottle to prepare the fluticasone furoate-vilanterol co-suspension for inhalation.

### EXAMPLE 8 Fluticasone furoate-vilanterol trifenatate co-suspension for inhalation and the method for preparing thereof

The components of the single vial and the amount of formulation of fluticasone furoate-vilanterol trifenatate co-suspension for inhalation are: **fluticasone furoate-vilanterol trifenatate co-suspension for inhalation (pH 6.5) with 0.015% (w/w) EDTA / 0.01% (w/w) polysorbate 80 prepared in the example.**

| component | single vial formulated amount | total amount |
|---|---|---|
| fluticasone furoate raw material drug | 0.17mg | 0.085 g |
| vilanterol trifenatate raw material drug | 0.068mg | 0.034g |
| polysorbate 80 | 0.2mg | 0.1g |
| EDTA | 0.3mg | 0.15 g |
| sodium chloride | 17mg | 8.5 g |
| water for injection | *q.s.* to 2 mL | *q.s.* to 1000 mL |

The preparation method:
(1) adding the formulated amount of excipients to 800 ml of water for injection, stirring to dissolve and making up to 1000 ml, to prepare a buffer solution, and adjusting pH to 6.5 by 0.1 M citric acid-sodium citrate buffer at pH 6.6;
(2) dispersing the fluticasone furoate raw material in 100 ml buffer solution by disperser at a dispersing speed of 9000 rpm for a dispersion time of 30 min, and then adding the vilanterol trifenatate raw material, continue dispersing by the disperser at 9000 rpm for 30 min, to obtain a dispersion of the two raw materials;
(3) mixing the dispersion of the raw materials with the remaining buffer solution and dispersing by the disperser at 9000 rpm for 30 min to prepare a co-suspension; and
(4) filling the above-mentioned suspension into a 2 ml LDPE bottle to prepare the fluticasone furoate-vilanterol co-suspension for inhalation.

### EXAMPLE 9 Fluticasone furoate-vilanterol trifenatate co-suspension for inhalation and the method for preparing thereof

The components of the single vial and the amount of formulation of fluticasone furoate-vilanterol trifenatate co-suspension for inhalation are: **fluticasone furoate-vilanterol trifenatate co-suspension for inhalation (pH 6.5) with 0.015% (w/w) EDTA / 0.03% (w/w) polysorbate 80 prepared in the example.**

| component | single vial formulated amount | total amount |
|---|---|---|
| fluticasone furoate raw material drug | 0.17mg | 0.085 g |
| vilanterol trifenatate raw material drug | 0.068mg | 0.034g |
| polysorbate 80 | 0.6mg | 0.3g |
| EDTA | 0.3mg | 0.15 g |
| sodium chloride | 17mg | 8.5 g |
| water for injection | *q.s.* to 2 mL | *q.s.* to 1000 mL |

The preparation method:
(1) adding the formulated amount of excipients to 800 ml of water for injection, stirring to dissolve and making up to 1000 ml, to prepare a buffer solution, and adjusting pH to 6.5 by 0.1 M citric acid-sodium citrate buffer at pH 6.6;
(2) dispersing the fluticasone furoate raw material in the above 100 ml buffer solution by disperser at a dispersing speed of 9000 rpm for a dispersion time of 30 min, and then adding the vilanterol trifenatate raw material, continue dispersing by the disperser at 9000 rpm for 30 min, to obtain a dispersion of the two raw materials;
(3) mixing the dispersion of the raw materials with the remaining buffer solution and dispersing by the disperser at 9000 rpm for 30 min to prepare a co-suspension; and
(4) filling the above-mentioned suspension into a 2 ml LDPE bottle to prepare the fluticasone furoate-vilanterol co-suspension for inhalation.

### EXAMPLE 10 Fluticasone furoate-vilanterol trifenatate co-suspension for inhalation and the method for preparing thereof

The components of the single vial and the amount of formulation of fluticasone furoate-vilanterol trifenatate co-suspension for inhalation are: **fluticasone furoate-vilanterol trifenatate co-suspension for inhalation (pH 5.0) with 0.01% (w/w) EDTA / 0.02% (w/w) polysorbate 80 prepared in the example.**

| component | single vial formulated amount | total amount |
|---|---|---|
| fluticasone furoate raw material drug | 0.17mg | 0.085 g |
| vilanterol trifenatate raw material drug | 0.068mg | 0.034g |
| polysorbate 80 | 0.4mg | 0.2g |
| EDTA | 0.2mg | 0.1g |
| sodium chloride | 17mg | 8.5 g |
| water for injection | *q.s.* to 2 mL | *q.s.* to 1000 mL |

The preparation method is:
(1) adding the formulated amount of excipients to 800 ml of water for injection, stirring to dissolve and making up to 1000 ml, to prepare a buffer solution, and adjusting pH to 5.0 by 0.1 M citric acid-sodium citrate buffer at pH 6.6;
(2) dispersing the fluticasone furoate raw material in 100 ml buffer solution by disperser at a dispersing speed of 9000 rpm for a dispersion time of 30 min, and then adding the vilanterol trifenatate raw material, continue dispersing by the disperser at 9000 rpm for 30 min, to obtain a dispersion of the two raw materials;
(3) mixing the dispersion of the raw materials with the remaining buffer solution and dispersing by the disperser at 9000 rpm for 30 min to prepare a co-suspension; and
(4) filling the above-mentioned suspension into a 2 ml LDPE bottle to prepare the fluticasone furoate-vilanterol co-suspension for inhalation.

### TEST EXAMPLE 3 Comparison of related substances for stability at rest of co-suspension with different pH and EDTA amounts

The samples prepared in Examples 4-10 above were placed at 60° C for 10 days to conduct a high-temperature influence factor test. The stability of the samples was compared based on the results of the relevant substances in the influence factor test, as shown in Table 3 below.

**Table 3**

| No. | pH | EDTA | polysorbate 80 | related substance % | fluticasone furoate | | vilanterol trifenatate | |
|---|---|---|---|---|---|---|---|---|
| | | | | | T=0 | T=10d 60°C | T=0 | T=10d 60°C |
| Example 4 | 3.5 | 0.005 | 0.01 | total impurity | 0.11 | 0.17 | 0.29 | 0.45 |
| | | | | maximum single impurity | 0.06 | 0.06 | 0.08 | 0.20 |
| Example 5 | 3.5 | 0.015 | 0.01 | total impurity | 0.10 | 0.19 | 0.25 | 0.33 |
| | | | | maximum single impurity | 0.05 | 0.06 | 0.10 | 0.19 |
| Example 6 | 3.5 | 0.015 | 0.03 | total impurity | 0.09 | 0.17 | 0.26 | 0.44 |
| | | | | maximum single impurity | 0.04 | 0.05 | 0.09 | 0.21 |
| Example 7 | 6.5 | 0.005 | 0.01 | total impurity | 0.15 | 0.21 | 0.23 | 15.3 |
| | | | | maximum single impurity | 0.05 | 0.07 | 0.09 | 7.94 |
| Example 8 | 6.5 | 0.015 | 0.01 | total impurity | 0.17 | 0.21 | 0.21 | 5.6 |
| | | | | maximum single impurity | 0.06 | 0.06 | 0.08 | 2.37 |
| Example 9 | 6.5 | 0.015 | 0.03 | total impurity | 0.18 | 0.23 | 0.22 | 7.9 |
| | | | | maximum single impurity | 0.06 | 0.07 | 0.08 | 3.61 |
| Example 10 | 5.0 | 0.01 | 0.02 | total impurity | 0.19 | 0.25 | 0.29 | 0.86 |
| | | | | maximum single impurity | 0.05 | 0.06 | 0.07 | 0.23 |

According to the test data in Table 3 above, it can be seen that pH value has a significant impact on the stability of fluticasone furoate-vilanterol trifenatate co-suspension. When the pH is 6.5, the high-temperature stability of vilanterol trifenatate in fluticasone furoate-vilanterol trifenatate co-suspension is significantly reduced, and the content of impurities has increased significantly. Therefore, the appropriate pH range for the fluticasone furoate-vilanterol trifenatate co-suspension is 3.5-5.0, which is consistent with the conclusion of test example 2. The optimal EDTA amount is determined to be (0.015% w/w), and the optimal amount of polysorbate 80 is 0.01% (0.01% w/w), which provides the best stability of the co-suspension.

### TEST EXAMPLE 4 Comparison of related substances before and after sterilization of raw material drugs

This test example conducted a series of experiments to clarify the effects of sterilization temperature and duration on the related substances of fluticasone furoate and vilanterol trifenatate raw material drugs.

Moist heat sterilization and dry heat sterilization were used to treat fluticasone furoate and vilanterol trifenatate for a certain period of time. Sample 1 was sterilized under moist heat sterilization at 120°C for 8 minutes, sample 2 was sterilized under moist heat sterilization at 121°C for 15 minutes, sample 3 was sterilized under dry heat sterilization at 110°C for 2 hours, and sample 4 was sterilized under dry heat sterilization at 120°C for 2 hours. After sterilization, the related substances of samples were analyzed, as shown in Table 4 below.

**Table 4**

| Related Substance % | | before sterilization | 120°C×8min moist heat | 121°C×15min moist heat | 110°C×2h dry heat | 120°C×2h dry heat |
|---|---|---|---|---|---|---|
| fluticasone furoate | total impurity | 0.24 | 1.0 | 2.8 | NA | 0.20 |
| | maximum single impurity | 0.06 | 0.45 | 1.57 | NA | 0.06 |
| vilanterol trifenatate | total impurity | 0.14 | 7.0 | 15.8 | 0.26 | 0.58 |
| | maximum single impurity | 0.07 | 4.4 | 11.0 | 0.13 | 0.25 |

According to the test data in Table 4 above, it can be seen that both fluticasone furoate and vilanterol trifenatate raw material drugs are not tolerant to moist heat sterilization. However, fluticasone furoate can tolerate dry heat sterilization at 120°C for 2 hours, while vilanterol trifenatate shows an increase in total impurity and single maximum impurity after 2 hours at 110°C, which is within an acceptable range.

### TEST EXAMPLE 5 Preparation and stability study of suspension for raw material drugs after dry heat sterilization

Test Example 4 shows that fluticasone furoate can tolerate dry heat sterilization, and the related substances of vilanterol trifenatate partially increase after dry heat sterilization. The related substances of fluticasone furoate and vilanterol trifenatate after sterilization and prepared as a co-suspension of raw material drugs with the determined EDTA amount (0.015%) and polysorbate 80 amount (0.01%) were analyzed, and the stability of different pH ranges (3.5, 4.0, 4.5, 5.0) was studied again for 1-month acceleration and at high temperature for 10 days. The results are shown in Table 5 below.

**Table 5**

| different pH | related substance % | fluticasone furoate | | | vilanterol trifenatate | | |
|---|---|---|---|---|---|---|---|
| | | T=0 | T=30d 40°C | T=10d 60°C | T=0 | T=30d 40°C | T=10d 60°C |
| 3.5 | total impurity | 0.22 | 0.20 | 0.22 | 0.68 | 0.81 | 0.92 |
| | maximum single impurity | 0.08 | 0.07 | 0.08 | 0.13 | 0.33 | 0.38 |
| 4.0 | total impurity | 0.25 | 0.21 | 0.21 | 0.67 | 0.76 | 0.86 |
| | maximum single impurity | 0.08 | 0.07 | 0.08 | 0.19 | 0.34 | 0.34 |
| 4.5 | total impurity | 0.22 | 0.28 | 0.23 | 0.63 | 0.75 | 1.10 |
| | maximum single impurity | 0.07 | 0.09 | 0.08 | 0.13 | 0.33 | 0.35 |
| 5.0 | total impurity | 0.26 | 0.24 | 0.24 | 0.73 | 0.98 | 1.30 |
| | maximum single impurity | 0.07 | 0.08 | 0.09 | 0.15 | 0.33 | 0.34 |

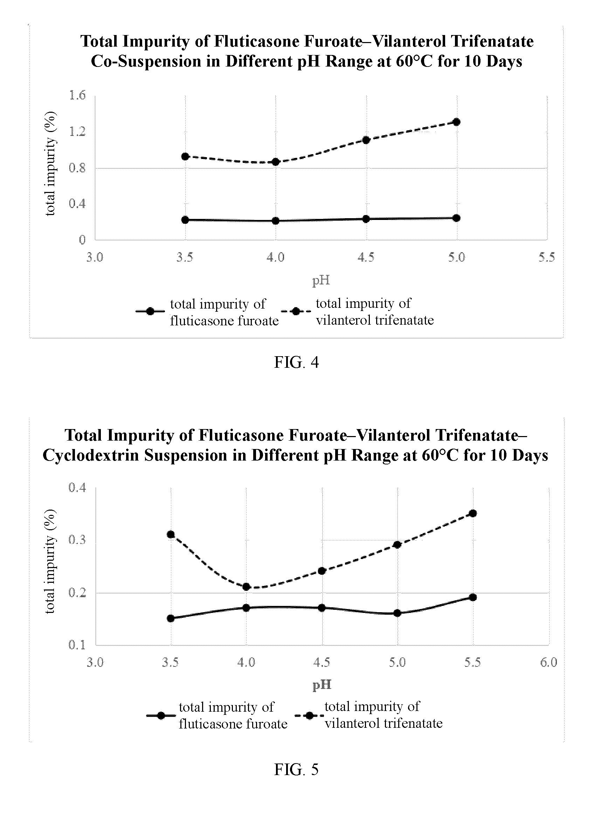

According to the test data in Table 5 above and Figure 4, it can be seen that fluticasone furoate has good stability in the pH range of 3.5-5.0, while the total impurity of vilanterol trifenatate is already high at day 0, and the total impurity increases significantly after being stored at 60°C for 10 days in the pH range of 4.5-5.0, indicating that the optimal pH value for vilanterol trifenatate is 3.5-4.0.

### EXAMPLE 11 Preparation of vilanterol trifenatate-cyclodextrin sterile inhalation solution (0.5% cyclodextrin)

0.075 g EDTA, 4.25 g NaCl, and 0.05 g polysorbate 80 were weighed and added into water for injection made up to 500 ml to prepare a buffer solution. 50 ml of the buffer solution was measured and 2.5 g of sulfobutyl-β-cyclodextrin was added to be dispersed and dissolved. 17mg of vilanterol trifenatate raw material drug was weighed and added into the solution above and dispersed at 9000 rpm for 30 minutes by a disperser to obtain a clear solution of vilanterol trifenatate. The solution and the remaining 450 ml buffer solution were dispersed at 9000 rpm for 30 minutes by a disperser to be mixed thoroughly. The solution remains clear without precipitation. The solution was filtered through a 0.45+0.22µm PVDF filter (manufacturer: Cobetter, model: SFM25DLHPVND4522P) to obtain a vilanterol trifenatate sterile inhalation solution.

### EXAMPLE 12 Preparation of vilanterol trifenatate-cyclodextrin sterile inhalation solution (1.5% cyclodextrin)

0.075 g EDTA, 4.25 g NaCl, and 0.05 g polysorbate 80 were weighed and added into water for injection made up to 500 ml to prepare a buffer solution. 150 ml of the buffer solution was measured and 7.5 g of sulfobutyl-β-cyclodextrin was added to be dispersed and dissolved. 17mg of vilanterol trifenatate raw material drug was weighed and added into the solution above and dispersed at 9000 rpm for 30 minutes by a disperser to obtain a clear solution of vilanterol trifenatate. The solution and the remaining 350 ml of the buffer solution were dispersed at 9000 rpm for 30 minutes by a disperser to be mixed thoroughly. The solution remains clear without precipitation. The solution was filtered through a 0.45+0.22µm PVDF filter (manufacturer: Cobetter, model: SFM25DLHPVND4522P) to obtain a vilanterol trifenatate sterile inhalation solution.

### EXAMPLE 13 Preparation of fluticasone furoate-vilanterol trifenatate-cyclodextrin sterile suspension

0.075 g EDTA, 4.25 g NaCl, and 0.05 g polysorbate 80 were weighed and added into water for injection made up to 500 ml to prepare a buffer solution. The solution was filtered through a 0.45+0.22µm PVDF filter (manufacturer: Cobetter, model: SFM25DLHPVND4522P) to obtain a sterile buffer solution. 150 ml of the buffer solution was measured and 7.5 g of sulfobutyl-β-cyclodextrin was added therein to be dispersed and dissolved, 17mg of vilanterol trifenatate raw material drug was weighed, and dispersed at 9000 rpm for 30 minutes by a disperser and filtered through a 0.45+0.22 µm PVDF filter (manufacturer: Cobetter, model: SFM25DLHPVND4522P), to obtain a clear solution of vilanterol trifenatate.

50 ml of the buffer solution was measured and 42.5 g of dry heat sterilized fluticasone furoate was weighed and added thereinto, and dispersed at 9000 rpm for 30 minutes by a disperser to obtain a fluticasone furoate suspension. The fluticasone furoate suspension, the vilanterol trifenatate solution and the remaining buffer were dispersed at 9000 rpm for 30 minutes to be mixed thoroughly, to obtain a fluticasone furoate-vilanterol trifenatate sterile inhalation suspension.

### TEST EXAMPLE 6 Comparison of related substances for stability at rest between vilanterol trifenatate single suspension and vilanterol trifenatate-cyclodextrin solution for inhalation

0.075 g EDTA, 4.25 g NaCl, and 0.05 g polysorbate 80 were weighed and added into water for injection made up to 500 ml to prepare a buffer solution. 50 ml of the buffer solution was measured and 17g of vilanterol trifenatate raw material drug was added into the solution above, and dispersed at 9000 rpm for 30 minutes by a disperser to obtain the dispersion of vilanterol trifenatate. The suspension and the remaining 450 ml buffer solution were dispersed at 9000 rpm for 30 minutes by a disperser to be mixed thoroughly to obtain the vilanterol trifenatate single suspension.

Vilanterol trifenatate-cyclodextrin solution for inhalation was prepared according to the formulation and process of Example 11, and the stability of the two was compared based on the content and related substances under high-temperature influence factors. The results are shown in Table 6.

**Table 6**

| stability comparison | | T=0 | T=10d 60°C |
|---|---|---|---|
| vilanterol trifenatate single suspension | content% | 100.3 | 95.6 |
| | total impurity | 0.23 | 2.60 |
| | maximum single impurity | 0.15 | 0.54 |
| vilanterol trifenatate-cyclodextrin solution for inhalation | content% | 102.0 | 102.4 |
| | total impurity | 0.16 | 0.59 |
| | maximum single impurity | 0.16 | 0.18 |

According to the test data in Table 6 above, it can be seen that compared to the vilanterol trifenatate single suspension, the inhalation solution prepared by mixing sulfobutyl-β-cyclodextrin and vilanterol trifenatate used in Example 11 has better stability at 60°C for 10 days, and higher stability assessed by content and related substances.

### TEST EXAMPLE 7 Comparison of related substances for stability at rest of fluticasone furoate-vilanterol trifenatate-cyclodextrin suspensions at different pH values

0.1M citrate acid-sodium citrate buffer solution at pH 3.0 and 0.1M citrate acid-sodium citrate buffer solution at pH 6.6 were prepared. The pH values of fluticasone furoate-vilanterol trifenatate-cyclodextrin suspensions prepared in example 13 were adjusted to 3.5, 4.0, 4.5, 5.0 and 5.5 respectively by the buffer solution above. The high-temperature influence factor test (60°C 10d) was conducted to study the stability under different pH ranges. The results are shown in Table 7.

**Table 7**

| different pH | related substance % | fluticasone furoate | | vilanterol trifenatate | |
|---|---|---|---|---|---|
| | | T=0 | T=10d 60°C | T=0 | T=10d 60°C |
| 3.5 | total impurity | 0.07 | 0.15 | 0.26 | 0.31 |
| | maximum single impurity | 0.04 | 0.05 | 0.15 | 0.17 |
| 4.0 | total impurity | 0.08 | 0.17 | 0.16 | 0.21 |
| | maximum single impurity | 0.04 | 0.05 | 0.16 | 0.16 |
| 4.5 | total impurity | 0.07 | 0.17 | 0.13 | 0.24 |
| | maximum single impurity | 0.04 | 0.06 | 0.13 | 0.17 |
| 5.0 | total impurity | 0.08 | 0.16 | 0.13 | 0.29 |
| | maximum single impurity | 0.04 | 0.06 | 0.13 | 0.16 |
| 5.5 | total impurity | 0.09 | 0.19 | 0.22 | 0.35 |
| | maximum single impurity | 0.04 | 0.06 | 0.13 | 0.18 |

According to the test data in Table 7 above and Figure 5, it can be seen that the total impurity of vilanterol trifenatate increases significantly at 60°C for 10 days when the pH is 3.5, and the total impurity of vilanterol trifenatate increases significantly at 60°C for 10 days when the pH is 5.5. Therefore, the optimal pH value for fluticasone furoate-vilanterol trifenatate-cyclodextrin co-suspension is 4.0-5.0.

### EXAMPLE 14 Preparation of fluticasone furoate-olodaterol hydrochloride sterile suspension for inhalation

0.075 g EDTA, 4.25 g NaCl, and 0.05 g polysorbate 80 were weighed and added into water for injection made up to 500 ml to prepare a buffer solution. The solution was filtered through a 0.45+0.22µm PVDF filter (manufacturer: Cobetter, model: SFM25DLHPVND4522P) to obtain a sterile buffer solution. 50 ml of the buffer solution was measured and 42.5mg of fluticasone furoate was weighed and added thereinto, and dispersed at 9000 rpm for 30 minutes by a disperser, to obtain a fluticasone furoate co-suspension.

34.2 mg of olodaterol hydrochloride was weighed and added into the remaining buffer solution, stirred with magnetic stirring at 300 rpm for 30 minutes, and then filtered through a 0.45+0.22µm PVDF filter (manufacturer: Cobetter, model: SFM25DLHPVND4522P) to obtain a sterile clear solution of olodaterol hydrochloride. Fluticasone furoate suspension and olodaterol hydrochloride solution were dispersed at 9000 rpm for 30 minutes by a dispenser to be mixed thoroughly, to obtain fluticasone furoate-olodaterol hydrochloride sterile co-suspension for inhalation.

### TEST EXAMPLE 8 Comparison of related substances for stability at rest of fluticasone furoate-olodaterol hydrochloride suspensions at different pH values

0.1M citrate acid-sodium citrate buffer solution at pH 3.0, 0.1M citrate acid-sodium citrate buffer solution at pH 6.6 and 1M sodium citrate solution were prepared. The pH values of fluticasone furoate-olodaterol hydrochloride suspensions were adjusted to 2.5, 3.0, 3.5, 4.0, 4.5, 5.0 and 5.5 respectively by the three solutions above. The high-temperature influence factor test (60°C 10d) was conducted to study the stability under different pH ranges. The results are shown in Table 8.

**Table 8**

| different pH | Related Substance % | fluticasone furoate | | olodaterol hydrochloride | |
|---|---|---|---|---|---|
| | | T=0 | T=10d 60°C | T=0 | T=10d 60°C |
| 2.5 | total impurity | 0.10 | 0.38 | 0.32 | 0.59 |
| | maximum single impurity | 0.05 | 0.09 | 0.09 | 0.17 |
| 3.0 | total impurity | 0.07 | 0.32 | 0.28 | 0.41 |
| | maximum single impurity | 0.05 | 0.09 | 0.07 | 0.08 |
| 3.5 | total impurity | 0.07 | 0.33 | 0.36 | 0.53 |
| | maximum single impurity | 0.04 | 0.09 | 0.18 | 0.16 |
| 4.0 | total impurity | 0.06 | 0.32 | 0.41 | 0.65 |
| | maximum single impurity | 0.04 | 0.11 | 0.16 | 0.26 |
| 4.5 | total impurity | 0.07 | 0.46 | 0.42 | 1.99 |
| | maximum single impurity | 0.05 | 0.10 | 0.18 | 1.03 |
| 5.0 | total impurity | 0.10 | 0.40 | 0.46 | 1.23 |
| | maximum single impurity | 0.05 | 0.10 | 0.16 | 0.72 |
| 5.5 | total impurity | 0.11 | 0.42 | 0.41 | 1.72 |
| | maximum single impurity | 0.06 | 0.08 | 0.16 | 1.11 |

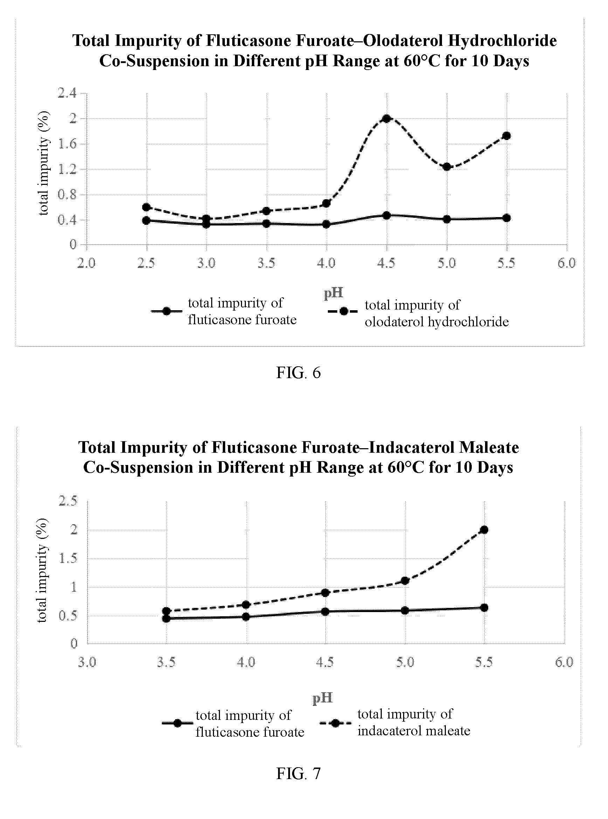

According to the test data in Table 8 above and Figure 6, it can be seen that, for the suspension formed by olodaterol hydrochloride and fluticasone furoate, the raw material fluticasone furoate has good stability in the pH range of 2.5-5.5, and the raw material olodaterol hydrochloride has good stability in the pH range of 2.5-4.0, especially the best stability at pH 3.0, while the total impurity content at 60°C for 10 days increases significantly when the pH value is greater than 4.0.

### EXAMPLE 15 Preparation of fluticasone furoate-indacaterol maleate sterile suspension

0.075 g EDTA, 4.25 g NaCl, and 0.05 g polysorbate 80 were weighed and added into water for injection made up to 500 ml to prepare a buffer solution, 39 mg of indacaterol maleate was weighed and added into 50 ml of the buffer solution above, after dispersed at 9000 rpm for 30 minutes by a disperser, the drug solution was homogenized through a high-pressure homogenizer for 3 cycles at 1000 bar, followed by 6 cycles at 2000 bar, to obtain a homogenized drug solution. The homogenized drug solution and the remaining buffer solution were dispersed at 9000 rpm for 30 minutes by a disperser to be mixed thoroughly. The mixed solution was filtered through a 0.22µm PVDF filter membrane (manufacturer: Cobetter, model: SFM25DLHPVND4522P) to obtain an indacaterol maleate sterile nanosuspension.

### EXAMPLE 16 Preparation of fluticasone furoate-indacaterol maleate sterile suspension

0.075 g EDTA, 4.25 g NaCl, and 0.05 g polysorbate 80 were weighed and added into water for injection made up to 500 ml, and filtered through a 0.45+0.22µm PVDF filter (manufacturer: Cobetter, model: SFM25DLHPVND4522P), to obtain a sterile buffer solution. 50 ml of the buffer solution was measured and 42.5mg of fluticasone furoate was added, and dispersed at 9000 rpm for 30 minutes by a disperser, to obtain a fluticasone furoate suspension.

50 ml of the buffer solution was measured and 39 mg of indacaterol maleate was weighed and added into the buffer solution above, after dispersed at 9000 rpm for 30 minutes by a disperser, the drug solution was homogenized through a high-pressure homogenizer for 3 cycles at 1000 bar, followed by 6 cycles at 2000 bar, to obtain the homogenized drug solution. The homogenized drug solution was filtered through a 0.22µm PVDF filter membrane (manufacturer: Cobetter, model: SFM25DLHPVND4522P) to obtain an indacaterol maleate nanosuspension.

Fluticasone furoate suspension, indacaterol maleate nanosuspension and the remaining buffer were dispersed at 9000 rpm for 30 minutes by a disperser to be mixed thoroughly, to obtain a fluticasone furoate-indacaterol maleate sterile co-suspension for inhalation.

### TEST EXAMPLE 9 Stability study for fluticasone furoate-indacaterol maleate co-suspension at different pH values

0.1M citrate acid-sodium citrate buffer solution at pH 3.0 and 0.1M citrate acid-sodium citrate buffer solution at pH 6.6 were prepared. The pH of fluticasone furoate-indacaterol maleate co-suspensions were adjusted to 3.5, 4.0, 4.5, 5.0 and 5.5 by the buffer solutions. The high-temperature influence factor test (60°C 10d) was conducted to study the stability under different pH ranges. The results are shown in Table 8.

**Table 8**

| different pH | Related Substance % | fluticasone furoate | | indacaterol maleate | |
|---|---|---|---|---|---|
| | | T=0 | T=5d 60°C | T=0 | T=5d 60°C |
| 3.5 | total impurity | 0.42 | 0.44 | 0.49 | 0.57 |
| | maximum single impurity | 0.04 | 0.06 | 0.11 | 0.17 |
| 4.0 | total impurity | 0.42 | 0.47 | 0.52 | 0.68 |
| | maximum single impurity | 0.04 | 0.07 | 0.08 | 0.23 |
| 4.5 | total impurity | 0.44 | 0.56 | 0.55 | 0.89 |
| | maximum single impurity | 0.04 | 0.10 | 0.11 | 0.37 |
| 5.0 | total impurity | 0.43 | 0.58 | 0.54 | 1.10 |
| | maximum single impurity | 0.04 | 0.11 | 0.11 | 0.53 |
| 5.5 | total impurity | 0.44 | 0.63 | 0.53 | 1.99 |
| | maximum single impurity | 0.04 | 0.12 | 0.12 | 1.20 |

According to the test data in Table 8 above and Figure 7, it can be seen that in the fluticasone furoate-indacaterol maleate co-suspension, the raw material fluticasone furoate has good stability in the pH range of 3.5-5.5, and there is no significant difference in single impurities and total impurity under various pH conditions; the raw material indacaterol maleate has good stability in the pH range of 3.5-4.5, especially the stability is the best at pH 3.5, and the total impurity content increases significantly at 60°C for 10 days when the pH value is greater than 4.5.

To sum up, the present application provides a once-a-day sterile suspension or co-suspension comprising an ultra-long-acting inhaled steroid and an ultra-long-acting β2 receptor agonists, which provides a reliable treatment scheme for patients who cannot use portable dry powder inhaler (DPI), metered dose inhaler (MDI) and soft mist inhaler (SMI), as well as children and critical patients.

Finally, it should be noted that the above examples are only used to illustrate the technical solution of the present invention and not to limit the scope of protection of the present invention. Although the present invention has been described in detail with reference to the preferred examples, those skilled in the art should understand that although the technical solution of the present invention can be modified or equivalently replaced, it does not depart from the essence and scope of the technical solution of the present invention.

## Claims

1. A pharmaceutical formulation comprising a combination of long-acting inhaled steroid and long-acting β2 receptor agonist, **characterized in that**: comprising:
the long-acting inhaled steroid drug is selected from fluticasone, or pharmaceutically acceptable salts; the long-acting β2 receptor agonist is selected from a group consisting of vilanterol, olodaterol, indacaterol, or any pharmaceutically acceptable salt, isomer, or hydrate thereof; and
a dosage form of the pharmaceutical formulation is a suspension or a co-suspension; and a pH of the pharmaceutical formulation is 2.5-6.0.

2. The pharmaceutical formulation according to claim 1, **characterized in that**: at least 90% of active agent particles by volume have an optical diameter of 7 µm or less; and at least 50% of the active agent particles by volume have an optical diameter of 5 µm or less.

3. The pharmaceutical formulation according to claim 1, **characterized in that**:
the long-acting inhaled steroid drug is selected from fluticasone and is fluticasone furoate; and
the long-acting β2 agonist is selected from one or more of vilanterol, olodaterol and indacaterol, wherein, the vilanterol is in a form of vilanterol trifenatate; the olodaterol is in a form of olodaterol hydrochloride; and the indacaterol is in a form of indacaterol maleate.

4. The pharmaceutical formulation according to claim 3, which is a nebulized inhalation suspension or co-suspension comprising fluticasone furoate and vilanterol trifenatate; wherein a daily inhalation dose of the fluticasone furoate is 25-1000 µg, and a daily inhalation dose of the vilanterol trifenatate is 10-100 µg.

5. The pharmaceutical formulation according to claim 4, **characterized in that**: the pharmaceutical formulation further comprises sulfobutyl-β-cyclodextrin; and the concentration of sulfobutyl-β-cyclodextrin in the pharmaceutical formulation is 5-500 mg/mL.

6. The pharmaceutical formulation according to claim 3, which is a nebulized inhalation suspension or co-suspension comprising fluticasone furoate and olodaterol hydrochloride; wherein a daily inhalation dose of the fluticasone furoate is 25-1000 µg, and a daily inhalation dose of the olodaterol hydrochloride is 1-100 µg.

7. The pharmaceutical formulation according to claim 3, which is a nebulized inhalation suspension or co-suspension comprising fluticasone furoate and indacaterol maleate; wherein a daily inhalation dose of the fluticasone furoate is 25-500 µg, and a daily inhalation dose of the indacaterol maleate is 20-200 µg.

8. The pharmaceutical formulation according to any one of claims 1-7, further comprising one or more of isotonic agents, buffering agents, excipients, stabilizers, and chelating agents.

9. The method for preparing the pharmaceutical formulation according to any one of claims 1-7, **characterized by** comprising the following steps:
(1) adding formulated amounts of excipients to water for injection and stirring to dissolve, adjusting the pH with buffer solution, and preparing a sterile buffer solution by filtering through a 0.22 µm filter membrane;
(2) dispersing a raw material of the long-acting inhaled steroid sterilized by dry heat in the aforementioned 10% volume buffer solution by a dispenser, to obtain a dispersion of the raw material of the long-acting inhaled steroid; and adding the β2 receptor agonist to a certain amount of buffer solution, and after specific processing, filtering through a 0.22 µm membrane, to obtain a dispersion of β2 receptor agonist;
(3) mixing the dispersions of the two raw materials with a remaining buffer solution, and dispersing with a disperser, to prepare a sterile suspension or co-suspension; and
(4) filling the suspension into a low-density polypropylene (LDPE) bottle to prepare a sterile suspension or co-suspension for inhalation.

10. Use of a pharmaceutical formulation according to any one of claims 1-7 in the preparation of a medicament for treating respiratory diseases; wherein the respiratory diseases comprise but are not limited to chronic obstructive pulmonary disease (COPD) and all types of asthma.
